Europäisches Patentamt

European Patent Office

Office européen des brevets

(11) Numéro de publication: **0 0 1 5 802**
**B1**

(12) **FASCICULE DE BREVET EUROPEEN**

(45) Date de publication du fascicule du brevet:
30.05.84

(51) Int. Cl.³: **C 07 D 277/72**

(21) Numéro de dépôt: 80400231.9

(22) Date de dépôt: **19.02.80**

(54) **Procédé de purification du mercaptobenzothiazole.**

(30) Priorité: 06.03.79 FR 7905710

(43) Date de publication de la demande:
17.09.80 Bulletin 80/19

(45) Mention de la délivrance du brevet:
30.05.84 Bulletin 84/22

(84) Etats contractants désignés:
BE CH DE FR GB IT LU NL SE

(56) Documents cités:
EP - A - 0 000 464

(73) Titulaire: **Société ATOCHEM, 12-16, Allée des Vosges, F-92400 Courbevoie (FR)**

(72) Inventeur: **Alicot, Michel Jean Camille, Route d'Arreau, F-65250 La Barthe de Neste (FR)**
Inventeur: **Tignol, Adrien Paul Noel, 15 rue des Pyrénées, F-31210 Montrejeau (FR)**

(74) Mandataire: Rochet, Michel, ATOCHEM Service
PROPRIETE INDUSTRIELLE Tour Manhattan Cédex 21,
F-92091 Paris la Défense (FR)

ACTORUM AG

## Description

Le mercaptobenzothiazole est connu dans l'industrie de transformation des élastomères pour son utilisation comme accélérateur de vulcanisation. Il est également une matière première importante dans la synthèse d'accélérateurs de vulcanisation plus perfectionnés, adaptés aux problèmes particuliers que pose la fabrication d'articles aussi différents que, par exemple, les pneumatiques, les cables électriques, les semelles de chaussures, les joints d'isolation. Il entre aussi pour une large dans la synthèse de composés phytosanitaires.

La plupart des procédés de fabrication connus font appel à la réaction, en proportions appropriées, à haute température et haute pression, de l'aniline, du soufre et du sulfure de carbone. D'autres font appel, soit à la réaction de la thiocarbanilide, du sulfure de carbone et du soufre (brevet US n° 1712968 du 14.05.1929) soit à la réaction de l'orthochloronitrobenzène, de l'hydrogène sulfuré ou d'un sulfure alcalin et du sulfure de carbone (brevet US n° 1960205 du 22.05.1934, brevet polonais n° 86988 du 15.12.1976), soit encore à la réaction du benzothiazole et du soufre (brevet allemand n° 2551060 du 26.05.1976). Le produit de réaction obtenu dans de telles conditions n'est jamais directement utilisable tel quel. Il contient, en effet, des matières premières n'ayant pas réagi, par exemple de l'aniline, des sous-produits et intermédiaires tels que le benzothiazole, l'anilinobenzothiazole. Une purification soigneuse de produit brut de réaction est nécessaire.

De nombreux procédés de purification ont été proposés à ce jour. Ils font appel, fondamentalement, à trois techniques qui diffèrent principalement par les concentrations, l'ordre d'emploi, la nature des réactifs préconisés, les températures de traitement.

Le schéma de principe de la première technique est le suivant:

— solubilisation du produit de réaction en milieu alcalin (hydroxyde d'ammonium, hydroxyd de sodium, hydroxyde de calcium) précédée ou non d'un traitement en milieu acide minéral;

— séparation des impuretés insolubles par filtration;

— séparation des impuretés solubles après leur insolubilisation par oxydation et/ou extraction à l'acide d'un solvant;

— précipitation du mercaptobenzothiazole par action d'un acide minéral.

Les brevets US n° 1631871, 2658864, 2730528 et 3818025 et le brevet français n° 2135807 illustrent l'application, en totalité ou en partie, d'un tel processus.

Dans la deuxième technique, les impuretés sont extraites par traitement du produit de réaction par le sulfure de carbone ou une émulsion de sulfure de carbone et d'eau. Les brevets US n° 2090233, 3030373 et 3031073 illustrent ce mode de réalisation.

Bien qu'ils soient actuellement industrialisés, ces procédés ne sont pas satisfaisants et présentent chacun, en totalité ou en partie, les inconvénients suivants:

— Récupération difficile des matières premières n'ayant pas réagi et que l'on a le plus grand intérêt économique à recycler (aniline, tout particulièrement).

— Nécessité d'opérer à des concentrations peu élevées pour favoriser la précipitation des impuretés, avec pour conséquence des appareillages de grandes dimensions.

Pertes, par dégradation chimique, de mercaptobenzothiazole au cours des réactions d'oxydation destinées à insolubiliser les impuretés solubles en milieu alcalin.

— Pertes, par solubilisation dans le sulfure de carbone, de mercaptobenzothiazole ou recyclage inévitable d'une partie des impuretés si la quantité de sulfure de carbone est limitée. Inconvénient supplémentaire s'avoir à manipuler des quantités importantes de ce réactif hautement inflammable.

— Enfin, et c'est peut-être l'inconvénient majeur de ces procédés, l'obligation d'avoir à traiter avant leur rejet de grands volumes d'effluents aqueux contenant de fortes charges polluantes. Ces traitements sont difficiles et coûteux. Les processus d'oxydation généralement applqués ne conduisent pas aux molécules simples souhaitées d'azote, de gaz carbonique et d'anhydride sulfureux, mais à un taux non acceptable de molécules solubles qui ne sont dégradées que par un traitement biologique complémentaire aggravant lourdement le coût des unités de production et des prix de revient.

Une troisième technique met à profit la propriété de certains solvants de solubiliser les impuretés contenues dans le produit brut de réaction, le mercaptobenzothiazole étant lui-même insoluble dans ces solvants. Cette technique s'avère très avantageuse par rapport aux précédentes en raison de sa simplicité et du rendement de purification qu'elle permet d'atteindre. Le tétrachloro-1,1,2,2 éthylène et le tétrachlorure de carbone sont, à ce titre, des solvants particulièrement efficaces (EP-A 00464).

On peut cependant considérer que malgré les progrès que cette technique apporte par rapport aux précédentes, elle nécessite l'introduction dans le processus de fabrication d'une matière première supplémentaire avec les conséquences que cela comporte: stockages supplémentaires, matériel de récupération.

Or, il a été trouvé, dans les services de la demanderesse, que l'on peut, à partir du produit brut résultant de la réaction de l'aniline, du soufre et du sulfure de carbone réagissant à haute pression et haute température suivant des procédés connus, obtenir du mercaptobenzothiazole avec un haut rendement de purification dans un grand état de pureté, au moyen d'une technique simplifiée qui met à produit le caractère solvant de l'un des réactifs de la réaction.

Le procédé selon l'invention pour la purification du mercaptobenzothiazole consiste en ce que le produit brut résultant de la réaction de l'aniline, du soufre et du sulfure de carbone suivant les procédés connus, est traité par de l'aniline, matière première de la réaction.

Il consiste plus particulièrement à ajouter au produit brut résultant de la réaction de l'aniline, du soufre et du sulfure de carbone, de l'aniline en quantité suffisante pour solubiliser les impuretés, soit directement dans le réacteur où a été effectuée la synthèse du mercaptobenzothiazole, à une température comprise entre 170° C et 300° C, avant de refroidir et de détendre à pression atmosphérique de telle façon à insolubiliser le mercaptobenzothiazole, de filter et de laver à l'aniline le mercaptobenzothiazole précipité, soit après détente à pression atmosphérique et dégazage, à une tempérture comprise entre 15° C et 184° C, avant de filtrer le mélange ainsi obtenu dont la température est telle que le mercaptobenzothiazole est insolubilisé et de laver avec de l'aniline le mercaptobenzothiazole précipité.

En vue de la réalisation du procédé selon l'invention, il est avantageux d'opérer comme suit:

– Addition de l'aniline au produit de la réaction en quantité suffisante pour solubiliser les impuretés.

– Filtration et lavage à l'aniline du mercaptobenzothiazole insolubilisé.

– Recyclage de la phase liquide du milieu de la purification.

Etape 1

L'addition de l'aniline peut être réalisée suivant deux variantes:

1.1. Directement dans le réacteur de synthèse, préférentiellement en fin de réaction, par tout moyen connu (comme par exemple une pompe doseuse heute pression). L'introduction en tête du réacteur n'est pas conseillée bien que cela soit possible, en raison des réactions secondaires de l'aniline en fort excès avec les divers composants du mélange réactionnel. La température à laquelle est réalisée l'addition d'aniline est comprise entre la température de réaction et la température de solidification du produit brut, c'est-à-dire entre 170° C et 300° C. Préférentiellement l'intervalle choisi est 180° C–220° C.

Dans ce mode opératoire, la température de détente à la pression atmosphérique peut être nettement inférieure à la température de solidification du mélange réactionnel. Elle n'est fonction que de la quantité d'aniline ajoutée en liaison avec la valeur de la solubilité du mercaptobenzothiazole dans ce solvant.

1.2. L'addition d'aniline peut être effectuée suivant une autre variante: par mélange avec le produit brut de réaction, après détente à la pression atmosphérique; préférentiellement les produits gazeux tel que l'hydrogène sulfuré sont préalablement séparés.

La température de mélange est dans ce cas comprise entre la température ambiante et la température d'ébulition de l'aniline, soit entre 15° C et 184° C.

Avant de réaliser la filtration, et de manière à ce que la solubilisation des impuretés et le lavage soitent efficaces, il est préférable qu'au sein du mélange réactionnel dilué à l'aniline et préférentiellement amené à la température ambiante, les particules de mercaptobenzothiazole insolubilisées soient d'une granulométrie fine. Bien qu'elle ne soit pas limitative, une valeur inférieure à 100 microns est considérée comme satisfaisante. On obtient ce résultat, aisément, par tout moyen connu.

Etape 2: Filtration – Lavage

La filtration et le lavage à l'aniline du mercaptobenzothiazole précipité est du domaine de l'homme de l'art ordinaire. La quantité d'aniline utilisée est liée à l'efficacité des moyens technologiques mis en œuvre. L'élimination de l'aniline contenue dans le gâteau de filtration essoré après lavage peut être effectuée suivant la technique de l'entraînement à la vapeur d'eau ou de l'évaporation sous vide. On préfère avoir recours à cette dernière. Réalisée en continu, elle simplifie la récupération de l'aniline et surtout évite tout effluent aqueux.

Etape 3: Recyclage

Le recyclage des phases liquides est réalisé après concentration, par distillation d'aniline, jusqu'à un volume déterminé (lié à celui dans lequel on choisit d'opérer la solubilisation des impuretés) et après la purge d'un volume calculé de manière à ce que soit éliminé par unité de temps un poids d'impureté égal à celui apporté par le produit de réaction.

Ceci suppose que préalablement, dans une période de mise en régime, le recyclage des phases liquides soit effectué un certain nombre de fois sans purge. De cette manière le taux d'impuretés devenant suffisamment élevé par rapport à celui du mercaptobenzothiazole solubilisé, les pertes en ce dernier produit dans la purge deviennent économiquement acceptables. Bien que le procédé avec les trois étapes ci-dessus soit le procédé préférentiel, on ne sortirait pas du cadre de l'invention en s'abstenant de recycler les phases liquides.

Le procédé de l'invention permet de récupérer sans difficultés les matières premières n'ayant pas réagi et les produits secondaires valorisables pouvant se trouver dans le produit de réaction: aniline et benzothiazole en particulier. Cette récupération se fait d'une part lors de la concentration des phases liquides avant recyclage, d'autre part par distillation de la fraction purgée.

Par la possibilité qu'il offre de réaliser la purifction du mercaptobenzothiazole sans avoir recours à une phase aqueuse, le procédé de l'invention supprime pratiquement les problèmes posés par le traitement des rejets de ce type d'installation industrielle.

L'exemple suivant illustre l'invention sans la limiter.

Exemple

Cet exemple décrit la purification du mercaptobenzothiazole au moyen de l'aniline après mise en régime (voir ci-dessus étape 3). La mise en état d'équilibre du milieu de purification est réalisée par une série de recyclage de la totalité des impuretés à éliminer, de manière à ce que le taux d'impuretés soit suffisamment élevé.

Dans un réacteur équipé d'un agitateur émulseur, d'une sonde de température, on introduit 1000 grammes de phase liquide provenant d'une opération antérieure de purification du mercaptobenzothiazole, de composition: 50,7% en aniline, 1,24% en benzothiazole, 18,8% en mercaptobenzothiazole et 29,25% en sous-produits divers.

Sous vive agitation, on introduit, prélevés à la température de 180° C à la sortie d'un réacteur de synthèse de mercaptobenzothiazole et après dégazage de l'hydrogène sulfuré, 400 grammes de mélange réactionnel provenant de la réaction du soufre, de l'aniline et du sulfure de carbone de composition: 2,75% en aniline, 5% en benzothiazole, 83,25% en mercaptobenzothiazole et 9% en sous-produits divers.

On agite énergiquement pendant environ une heure à la température de 20 à 25° C. La granulométrie des particules de mercaptobenzothiazole insolubilisée est de l'ordre de 40 à 60 microns. On filtre. Le produit sur filtre est lavé avec 100 grammes d'aniline en plusieurs fois et essoré.

Le gâteau obtenu d'un poids d'environ 700 grammes est soumis à évaporation sous vide (environ 15 à 20 mm Hg) à la température de 100 à 110° C, sous léger balayage d'azote. On recueille 329 grammes de mercaptobenzothiazole purifié de titre 98,8% de point de fusion (non corrigé) 177–180° C.

Le rendement de purification est de 97,6%.

Le filtrat issu de la filtration, après purge de 90 grammes, est réuni aux diverses fractions de lavage: après concentration à 1000 grammes par distillation sous pression réduite, il est recyclé dans une nouvelle opération de purification.

La purge, dont la teneur en mercaptobenzothiazole est de 8,9%, valeur qui représente environ 8 grammes soit 2,4% de la quantité de mercaptobenzothiazole introduit, est distillée sous vide de manière à récupérer l'aniline et le benzothiazole. Les indistillables sont soit éliminés soit recyclés en totalité ou en partie dans le réacteur de synthèse.

## Revendication

1. Procédé pour la purification du mercaptobenzothiazole caractérisé en ce que l'on ajoute de l'aniline au produit brut résultant de la réaction de l'aniline, du soufre et du disulfure de carbone, dans le réacteur où cette synthèse a été effectuée, à une température comprise entre 170° C et 300° C, l'aniline étant ajoutée en quantité suffisante pour solubiliser les impuretés, refroidit et détend à pression atmosphérique de telle façon à insolubiliser le mercaptobenzothiazole, filtre et lave à l'aniline le mercaptobenzothiazole précipité.

2. Procédé selon la revendication 1 dans lequel l'aniline est ajoutée à une température comprise entre 180° C et 220° C.

3. Procédé pour la purification du mercaptobenzothiazole caractérisé en ce que, après détente à la pression atmosphérique et dégazage du produit brut résultant de la réaction de l'aniline, du soufre et du disulfure de carbone, on mélange le produit brut dégazé avec de l'aniline en quantité suffisante pour solubiliser les impuretés, à une température comprise entre 15° C et 184° C, filtre le mélange ainsi obtenu dont la température est telle que le mercaptobenzothiazole soit insolubilisé et lave avec de l'aniline le mercaptobenzothiazole précipité.

4. Procédé selon chacune des revendications 1 et 3 dans lequel les particles de mercaptobenzothiazole du mélange dilué avec l'aniline et refroidi sont amenées à l'état de partiquels inférieurs à 100 microns avant d'effectuer la filtration.

5. Procédé selon chacune des revendicatons 1 et 3 caractérisé en ce que l'aniline utilisée pour la purification comprend de l'aniline de récupération obtenue, d'une part, par évaporation sous vide du mercaptobenzothiazole lavé à l'aniline, et, d'autre part, par concentration et distillation des filtrates de lavage.

## Patentansprüche

1. Verfahren zur Reinigung von Mercaptobenzthiazol, dadurch gekennzeichnet, dass man dem Rohprodukt aus der Umsetzung von Anilin, Schwefel und Kohlenstoffdisulfid in dem Reaktionsgefäss, in dem diese Synthese durchgeführt wurde, bei einer Temperatur zwischen 170° C und 300° C Anilin zugibt, wobei das Anilin in einer Menge zugegeben wird, die ausreicht, die Verunreinigung zu lösen, abkühlt und entspannt bei Atmosphärendruck auf die Weise, dass das Mercaptobenzthiazol unlöslich gemacht wird, filtriert und das ausgefällte Mercaptobenzthiazol mit Anilin wäscht.

2. Verfahren nach Anspruch 1, bei dem das Anilin bei eine Temperatur zwischen 180° C und 220° C zugegeben wird.

3. Verfahren zur Reinigung von Mercaptobenzthiazol, dadurch gekennzeichnet, dass man nach der Entspannung auf Atmosphärendruck und der Entgasung des aus der Reaktion von Anilin, Schwefel und Kohlenstoffdisulfid erhaltenen Rohproduktes, das entgaste Rohprodukte mit Anilin in einer Menge vermischt, die ausreicht, um die Verunreinigungen zu lösen bei einer Temperatur zwischen 15° und 184° C, das auf diese Weise erhaltene Gemisch filtriert, dessen Temperatur so ist, dass das Mercaptobenzthiazol unlöslich ist und das ausgefällte Mercaptobenzthiazol mit Anilin wäscht.

4. Verahren nach einem der Ansprüche 1 und 3, bei dem die mit Anilin verdünnten und abgekühlten Mercaptobenzthiazolpartikel des Gemisches in eine Teilchengrösse unterhalb 100μ überführt werden, ehe filtriert wird.

5. Verfahren nach einem der Ansprüche 1 und 3, dadurch gekennzeichnet, dass das für die Reinigung verwendete Anilin wiedergewonnenes Anilin ist, das man teilweise durch Vakuumverdampfung des mit Anilin gewaschenen Mercaptobenzthiazols und teilweise durch Konzentration und Destillation der Waschfiltrate erhält.

## Claims

1. Process for the purification of mercaptobenzothiazole, characterised by adding aniline to the crude product resulting from the reaction of aniline, sulphur and carbon disulphide, in the reactor where this synthesis has been effected, at a temperature between 170°C and 300°C, the aniline being added in sufficient quantity to solubilise the impurities, cooling and expanding to atmospheric pressure in such a manner as to insolubilise the mercaptobenzothiazole, filtering and washing the precipitated mercaptobenzothiazole with aniline.

2. Process according to claim 1, in which the aniline is added at a temperature between 180°C and 200°C.

3. Process for the purification of mertcaptobenzothiazole, characterised in that after expanding to atmospheric pressure and degassing the crude product resulting from the reaction of aniline, sulpur and carbon disulphide, the degassed crude product is mixed with the aniline in sufficient quantity to solubilse the impurities, at a temperature betwenn 15°C and 184°C, the mixture thus obtained, the temperature of which is such that the mercaptobenzothiazole is insolubilised, is filtered and the precipiated mercaptobenzotziazole is washed with aniline.

4. Process according to each of claims 1 and 3 in which the particles of mercaptobenzothiazole in the mixture diluted with aniline, and cooled, are brought into a condition in which the particles are smaller than 100 microns, before filtration is effected.

5. Process according to each of claims 1 and 3, characterised by the fact that the aniline used for the purification comprises recovered aniline, obtained, on the one hand, by evaporation under vacuum of the mercaptobenzothiazole washed whith aniline, and, on the other hand, by concentration and distillation of the washing filtrates.